# EUROPEAN PATENT APPLICATION

(11) **EP 2 469 500 A1**
(43) Date of publication of application: **27.06.2012**
(21) Application number: 10810026.4
(22) Date of filing: 20.08.2010
(51) Int. Cl.: G09B 23/28, A61B 17/04, A61B 19/00, G09B 9/00

(54) **SURGICAL TRAINING DEVICE**

(30) Priority: 20.08.2009 JP 2009191052
(71) Applicant: Nakamura, Shoichi, Higashichikuma-gun, Nagano 399-7502 (JP); ACP Japan Co. Ltd., Tokyo 113-0033 (JP)
(72) Inventor: NAKAMURA, Shoichi, Higashichikuma-gun, Nagano 399-7502 (JP)
(74) Representative: Gassner, Wolfgang
(86) International application number: PCT/JP2010/064055
(87) International publication number: WO 2011/021685

(57) **Abstract**

To provide a compact surgical training device for enabling skills of techniques to be improved efficiently and effectively before acquiring clinical experiences in surgeries with high degrees of difficulty, particularly, vascular anastomosis, the surgical training device 10 to perform simulations and practices of vascular anastomosis is provided with a first holder 110 that holds a first pseudo blood vessel BV1, a second holder 120 that holds a second pseudo blood vessel BV2 to be anastomosed to the first pseudo blood vessel BV1, and a support mount 100 that supports the first and second holders 110, 120 so as to cause the entire surgical training device 10 to stand, while enabling the first and second pseudo blood vessels BV1, BV2 to be placed in respective arbitrary positions.

## Description

### Technical Field

The present invention relates to a surgical training device used in training in surgeries, particularly, vascular anastomosis.

### Background Art

In recent years, surgeries have made dramatic progress with remarkable technological innovation. With the progress, surgical techniques are continuously made complicated and specialized, and the experiences and skills of operators are more strongly demanded than ever before. One of the techniques significantly dependent on the ability of the operator includes vascular anastomosis.

As the surgery for conducting vascular anastomosis, for example, there is the coronary artery bypass operation performed on patients having ischemic heart diseases such as cardiac infarction. This is an operation for exposing the heart and anastomosing a bypass blood vessel to send blood from another blood vessel to the coronary artery with the occurrence of stenosis that is the cause of ischemia. The operation is recognized as a method for enabling the blood circulation reconstruction effect to be maintained for the long term.

Further, as well as the operation, there are lymph duct veinlet anastomosis on lymphatic edema, cerebrovascular anastomosis applied to part of cases of cerebral infarction, cerebral ischemia cases by Willis arterial cycle occlusive disease (moyamoya disease) and the like, etc.

Then, devices for training in suture techniques of the surgery have conventionally been disclosed as a surgical suture practice instrument (for example, see Patent Document 1).

### Prior Art Document

### Patent Document

Patent Document 1: Japanese Patent Publication No. S60-35150

### Summary of Invention

### Problems to be Solved by the Invention

In the surgical suture practice instrument as disclosed in above-mentioned Patent Document 1, the subject of suture is a cloth member, is not able to contribute to training in anastomosis for anastomosing the blood vessels in actual surgeries, and has the problem of not being actual simulations, practices and training in surgeries with high degrees of difficulty such that the mutual angles are different between the blood vessel to be sutured and the blood vessel to suture.

Accordingly, to improve skills in the surgery, particularly, vascular anastomosis as described above, acquiring actual surgery experiences is only the method, and under present circumstances, it has been difficult to improve skills efficiently and effectively.

The present invention was made with attention directed toward the above-mentioned circumstances in the medical educational field, and it is an object of the invention to provide a compact surgical training device for enabling skills of techniques to be improved efficiently and effectively before acquiring clinical experiences in surgeries with high degrees of difficulty, particularly, vascular anastomosis.

### Means for Solving the Problem

To attain the aforementioned object, the present invention provides a surgical training device to perform simulations and practices of vascular anastomosis, which is characterized by having a support mount of the surgical training device, a first holder disposed on the support mount to hold a first pseudo blood vessel, and a second holder disposed on the support mount to hold a second pseudo blood vessel to be anastomosed to the first pseudo blood vessel, where the first holder holds the first pseudo blood vessel to enable a front end portion of the first pseudo blood vessel to be placed in an arbitrary position in the second pseudo blood vessel held by the second holder and in an arbitrary direction with respect to the axis direction of the second pseudo blood vessel.

Herein, in a first usage mode of the surgical training device of the invention, the first holder is characterized by having a hold member, formed on the top surface of the support mount, having a single or a plurality of grooves with different diameters to fix and hold the first pseudo blood vessel.

Further, in a second usage mode of the surgical training device of the invention, the first holder is characterized by being a multijoint movable holder that is coupled to the support mount and that enables the first pseudo blood vessel to be shifted to an arbitrary position while holding the first pseudo blood vessel in the axis direction of the first pseudo blood vessel.

Then, in the first and second usage modes of the surgical training device, the second holder is characterized by being a multijoint movable holder that is coupled to the support mount and that enables the second pseudo blood vessel to be shifted arbitrarily while holding the second pseudo blood vessel in the blood vessel axis direction of the second pseudo blood vessel.

Further, in a third usage mode of the surgical training device of the invention, it is also possible to use the first holder and the second holder as fix holders formed in an upper portion of the support mount to fix and hold the first pseudo blood vessel and the second pseudo blood vessel in the axis direction of each blood vessel, respectively.

Herein, the movable holder is characterized by having a flexible arm portion comprised of a structure for alternately coupling a plurality of connector members each in the shape of a dumbbell having balls at opposite ends of a cylinder, and a plurality of cylindrical bodies into which the balls of the connector members are fitted to hold the balls rotatably.

### Advantageous Effect of the Invention

According to the surgical training device of the invention, it is possible to reproduce environments closer to clinical experiences with a simplified configuration, and to contribute to actual simulations, practices and training in surgeries with high degrees of difficulty such that mutual angles are different between the blood vessel to be sutured and the blood vessel to suture before acquiring actual surgical experiences.

Further, since the structure is simplified, the device is extremely compact and inexpensive, and is easy to introduce to the medical educational field.

### Brief Description of Drawings

FIG. 1 is an entire schematic view of a surgical training device according to a first usage mode of the invention;
FIG. 2 is an exploded perspective view showing outlines of a support mount and a first holder in the surgical training device as shown in FIG. 1;
FIG. 3 is a schematic view showing an outline of a second holder in the surgical training device as shown in FIG. 1;
FIG. 4 contains schematic views showing an outline of a flexible arm portion in the surgical training device as shown in FIG. 1;
FIG. 5 is a schematic view showing an example of a hold member provided with a plurality of grooves;
FIG. 6 is a schematic view showing an example of end-side anastomosis in vascular anastomosis;
FIG. 7 is an entire schematic view of a surgical training device according to a second usage mode of the invention;
FIG. 8 is a schematic view showing an outline of a first holder in the surgical training device as shown in FIG. 7;
FIG. 9 is a schematic view showing an example of end-end anastomosis in vascular anastomosis;
FIG. 10 is an entire schematic view of a surgical training device according to a third usage mode of the invention;
FIG. 11 is an exploded perspective view showing outlines of a support mount and a fix hold portion in the surgical training device as shown in FIG. 10;
FIG. 12 is a schematic view showing an example in which a hold member is not used in the third usage mode of the invention;
FIG. 13 is a schematic view showing another example of a pressing plate; and
FIG. 14 contains schematic views illustrating the method for using the pressing plate of FIG. 13.

### Description of Embodiments

Embodiments of the invention will specifically be described below with reference to drawings.

### [First usage mode]

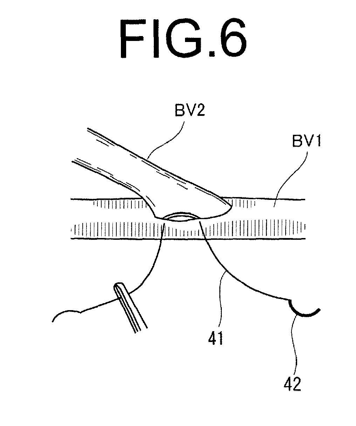

This usage mode describes the case of using a surgical training device of the invention in training for performing end-side anastomosis on pseudo blood vessels (anastomosing the end portion of one blood vessel to the side portion of another blood vessel; see FIG. 6).

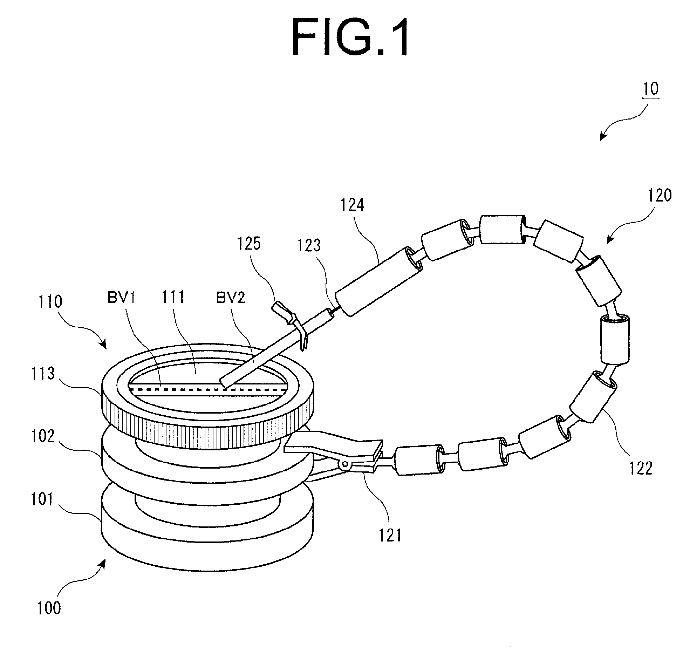

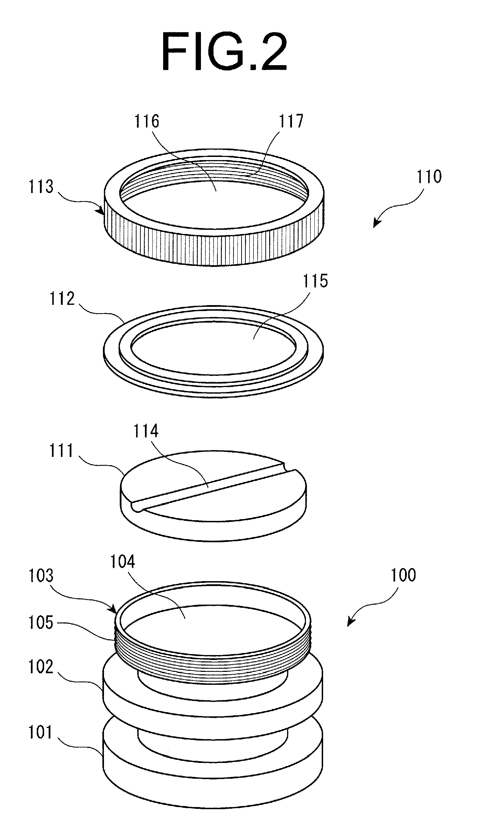

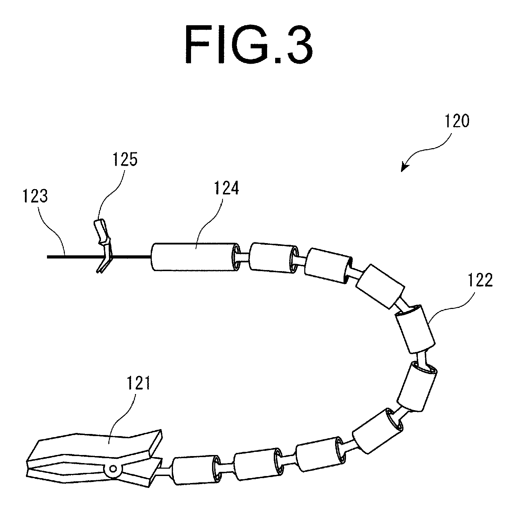

FIG. 1 is an entire schematic view of a surgical training device according to the first usage mode of the invention. FIG. 2 is an exploded perspective view showing outlines of a support mount and a first holder in the surgical training device as shown in FIG. 1, and FIG. 3 is a schematic view showing an outline of a second holder.

As shown in FIG. 1, the surgical training device 10 of this usage mode is comprised of a support mount 100, a first holder (fix holder) 110 on the top of the support mount 100 to hold a first pseudo blood vessel (or artificial blood vessel) BV1 in a fixed manner, and a second holder (movable holder) 120 that is coupled to the support mount 100 and that holds a second pseudo blood vessel (or artificial blood vessel) BV2 to be movable.

The support mount 100 has a support portion 101 that supports the entire training device 10 to cause the device to stand, a coupling portion 102 to couple the movable holder 120, and a fix holder installation portion 103 to attach the fix holder 110.

The support portion 101 has a structure capable of stably supporting the training device 10 without the device falling even when the movable holder 120 is moved to various positions and attitudes. More suitably, the bottom is provided with a nonslip material (not shown) comprised of silicone or the like.

The coupling portion 102 is a flange-shaped protrusion area to couple with a fix portion 121 of the movable holder 120, described later.

The fix holder installation portion 103 is comprised of a concave portion 104 provided on the upper face of the support mount 100 to fit the holder member 111 of the fix holder 110 thereinto, described below, and a screw portion 105 provided in the periphery of the concave portion 104.

The fix holder 110 is comprised of a hold member 111 to place the first pseudo blood vessel BV1, a fix portion 113 to fix the hold member 111 to the fix holder installation portion 103 of the support mount 100, and a pressing plate 112 that is positioned in between the hold member 111 and fix portion 113 to prevent the hold member 111 and the first pseudo blood vessel BV1 from being misaligned and the like.

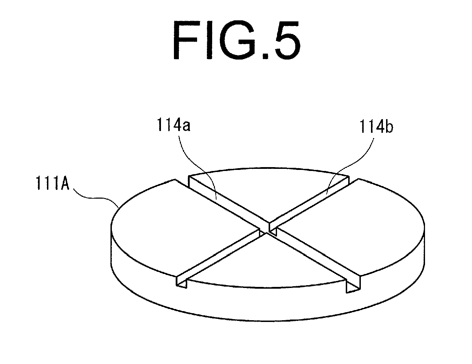

The hold member 111 is preferably comprised of a member having elasticity, and on its surface is formed a groove 114 to place the first pseudo blood vessel BV1. In addition, in FIGS. 1 and 2, the hold member 111 provided with only a single groove 114 is shown in the figures, but as shown in FIG. 5, the hold member maybe aholdmember 111A provided with a plurality of grooves (herein, two grooves as an example) 114a, 114b. In such a case, to support pseudo blood vessels (or artificial blood vessels) of various sizes, the grooves 114, 114b are configured to differ from each other in the groove width and the groove depth.

The pressing plate 112 is a plate-shaped member that presses the circumferential portion of the holdmember 111 and the endportion of the first pseudo blood vessel BV1, and has an opening 115.

The fix portion 113 has an opening 116 on the top, and in its inner circumference surface is formed a screw portion 117 that is screwed with the screw portion 105 of the support mount 100. Accordingly, while exposing the first pseudo blood vessel BV1 from the opening 116, the circumferential portion of the hold member 111 and the end portion of the first pseudo blood vessel BV1 are pressed together with the pressing plate 112, and are capable of being fixed to the support mount 100.

The movable holder 120 is comprised of a fix portion 121 to couple and fix the movable holder 120 to the coupling portion 102 of the support mount 100, a flexible arm portion 122 formed of a material or structure that is transformed flexibly, a hold shaft portion 123 that holds the second pseudo blood vessel BV2, and a connection portion 124 that connects between the flexible arm portion 122 and the hold shaft portion 123.

The fix portion 121 is suitably comprised of a clip as shown in FIGs. 1 and 3. It is preferable to provide the inside (the side coming into contact with the coupling portion 102 of the support mount 100) of the clip with a nonslip material such as silicone, not shown. The coupling portion 102 of the support mount 100 protruding in the shape of a fringe is pinched with such a clip, and the movable holder 120 is fixed to the support mount 100.

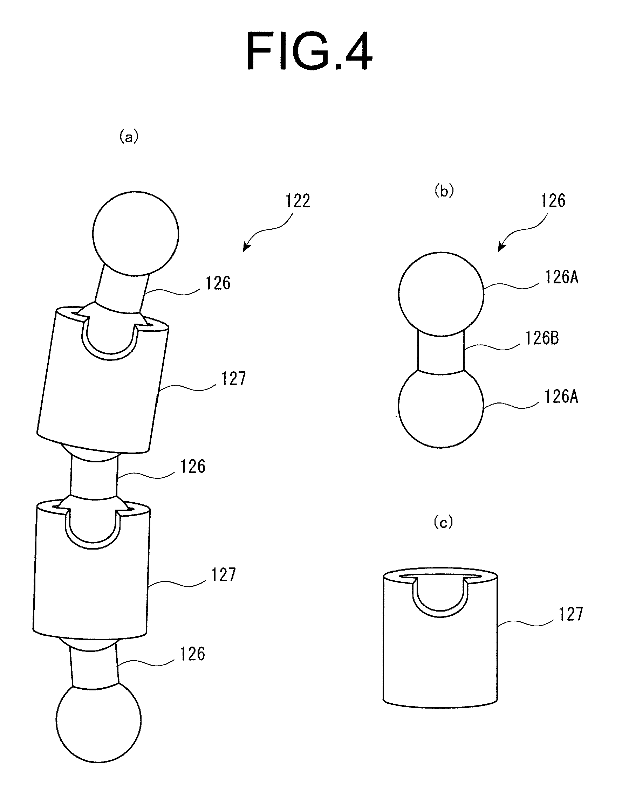

The flexible arm portion 122 is formed of a material or structure which can be transformed flexibly to place the second pseudo blood vessel BV 2 in an arbitrary position and an arbitrary angle, while being capable of maintaining the transform state. Herein, as an example, used is the structure that ball joints are coupled as shown in FIG. 3. FIG. 4 shows the details. As shown in the figure, herein, the flexible arm portion 122 is comprised of the ball joint structure that strongly fits a dumbbell-shaped connector member 126 comprised of balls 126A and a shaft 126B, and a cylindrical member 127 not to drop. Each of the balls 126A of the connector member 126 rotates inside the cylindrical member 127 freely, and it is thereby possible to bend and extend freely. Fine adjustments to the position and angle are also ease.

The hold shaft portion 123 is comprised of a shaft member which is inserted into a hole of the second pseudo blood vessel BV2 and thereby holds the second pseudo blood vessel BV2. Further, it is suitable to provide a clip 125 that fixes the second pseudo blood vessel BV2 in a predetermined insertion position of the hold shaft portion 123.

The connection portion 124 connects between the flexible arm portion 122 and the hold shaft portion 123. Since the thickness of the blood vessel significantly varies with sites (for example, aorta with a diameter of about 30 mm, coronary artery with a diameter of about 3mm, etc.), also pseudo blood vessels with different diameters are used in training. Accordingly, it is necessary to exchange the hold shaft portion 123 in accordance with the thickness of the pseudo blood vessel to use. Therefore, it is preferable that the connection portion 124 has a structure for enabling the hold shaft portion 123 to be exchanged with ease.

Described below is the case of performing vascular anastomosis training using the surgical training device 10 with the above-mentioned configuration.

First, the first pseudo blood vessel BV1 is fitted into the groove 114 provided in the hold member 111 of the fix holder 110, the hold member 111 is inserted in the concave portion 104, the pressing plate 112 is placed, and further, the fix portion 113 is attached to the fix holder installation portion 103. In addition, although not shown, it is suitable that a wire becoming a shaft to prevent misalignment is inserted into the first pseudo blood vessel BV1 disposed in the groove 114.

Meanwhile, the second pseudo blood vessel BV2 to be anatomized to the first pseudo blood vessel BV1 is inserted into the hold shaft portion 123 of the movable holder 120. At this point, the second pseudo blood vessel BV2 is pinched with the clip 125 together with the hold shaft portion 123 and fixed while protruding through the front end of the hold shaft portion 123 in a predetermined length. Then, the second pseudo blood vessel BV2 is placed in a position most suitable for anastomosis with the flexible arm portion 122 transformed flexibly of the second holder 120.

By this means, as shown in FIG. 6, the first pseudo blood vessel BV1 and the second pseudo blood vessel BV2 are anastomosed with a suture 41 and a surgical needle 42.

Thus, according to the aforementioned first usage mode, the support mount 100 and the fix holder 110 are integrated, the movable holder 120 that is transformed flexibly is further provided, and it is thereby possible to configure the surgical training device that is compact and easy to use, and to perform training in end-side anastomosis with ease.

### [Second usage mode]

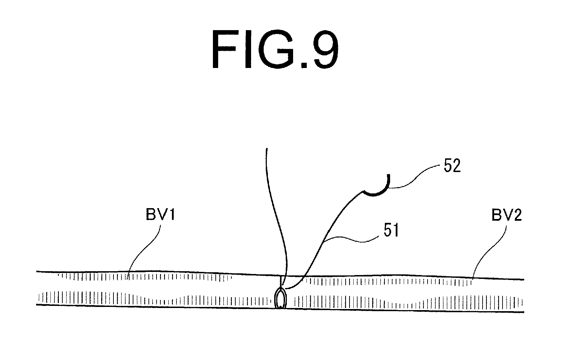

This usage mode describes the case of using a surgical training device of the invention in training for performing end-end anastomosis on pseudo blood vessels (anastomosing the end portion of one blood vessel to the end portion of another blood vessel; see FIG. 9).

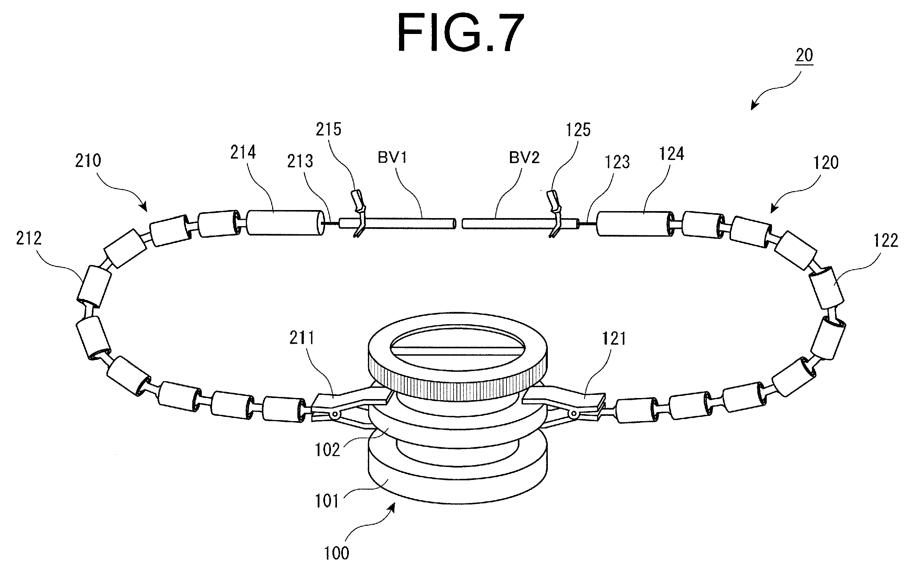

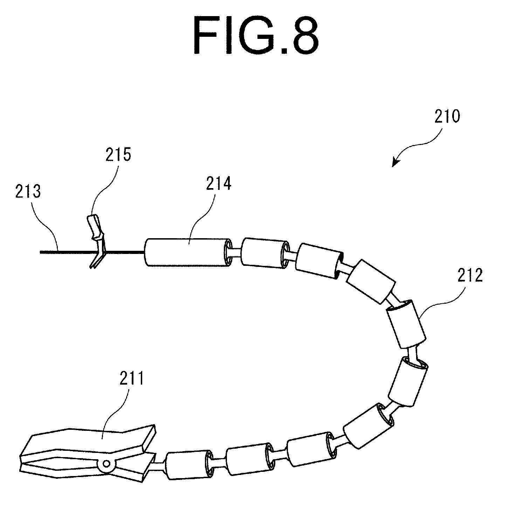

FIG. 7 is an entire schematic view of a surgical training device according to the second usage mode of the invention. FIG. 8 is a schematic view showing an outline of a first holder in the surgical training device as shown in FIG. 7.

In addition, the surgical training device 20 of this usage mode has the same configuration as in the first usage mode except a first holder 210. Therefore, the same portions as in the first usage mode shown in FIG. 1 are assigned the same reference numerals to omit specific descriptions thereof.

As shown in FIG. 7, the surgical training device 20 of this usage mode is comprised of the support mount 100, a first holder (first movable holder) 210 that is coupled to the support mount 100 and that holds the first pseudo blood vessel (or artificial blood vessel) BV1 to be movable, and the second holder (second movable holder) 120 that is similarly coupled to the support mount 100 and that holds the second pseudo blood vessel (or artificial blood vessel) BV2 to be movable.

Thus, by adding only the movable holder 210 to the surgical training device 10 as shown in the first usage mode, it is possible to configure the surgical training device 20 of this usage mode. The first movable holder 210 has the same structure as the second movable holder 120 (FIG. 3). In other words, as shown in FIG. 8, the first movable holder 210 is comprised of a fix portion 211 to couple and fix the first movable holder 210 to the coupling portion 102 of the support mount 100, a flexible arm portion 212 formed of a material or structure that is transformed flexibly, a hold shaft portion 213 that holds the first pseudo blood vessel BV1, and a connection portion 214 that connects between the flexible arm portion 212 and the hold shaft portion 213. The configuration of each of aforementioned portions is the same as the second movable holder 120 as shown in FIG. 3.

Described below is the case of performing vascular anastomosis training using the surgical training device 20 with the above-mentioned configuration.

First, the first pseudo blood vessel BV1 is inserted into the hold shaft portion 213 of the first movable holder. Further, the second pseudo blood vessel BV2 to be anatomized to the first pseudo blood vessel BV1 is inserted into the hold shaft portion 123 of the movable holder 120. At this point, the first pseudo blood vessel BV1 and the second pseudo blood vessel BV2 are pinched with the clips 215, 125 together with the hold shaft portions 213, 123 and fixed while protruding through the front ends of the hold shaft portions 213, 123 in predetermined lengths, respectively. Then, each of the pseudo blood vessels BV1, BV2 is placed in a position most suitable for anastomosis with the flexible arm portion 212 or 122 transformed flexibly of the first holder 210 or second holder 120, respectively.

By this means, as shown in FIG. 9, the first pseudo blood vessel BV1 and the second pseudo blood vessel BV2 are anastomosed with a suture 51 and a surgical needle 52.

Thus, according to the aforementioned second usage mode, the support mount 100 is provided two movable holders 210, 120 transformed flexibly, and it is thereby possible to configure the surgical training device that is compact and easy to use, and to perform training in end-end anastomosis with ease.

### [Third usage mode]

As in the second usage mode, this usage mode describes the case of using a surgical training device of the invention in training for performing end-end anastomosis on pseudo blood vessels. In addition, herein, shown is an example of holding the pseudo blood vessels with fix holders, instead of holding the pseudo blood vessels with movable holders as in the second usage mode.

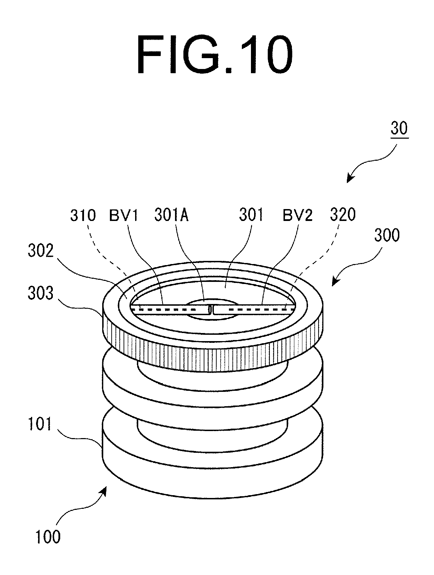

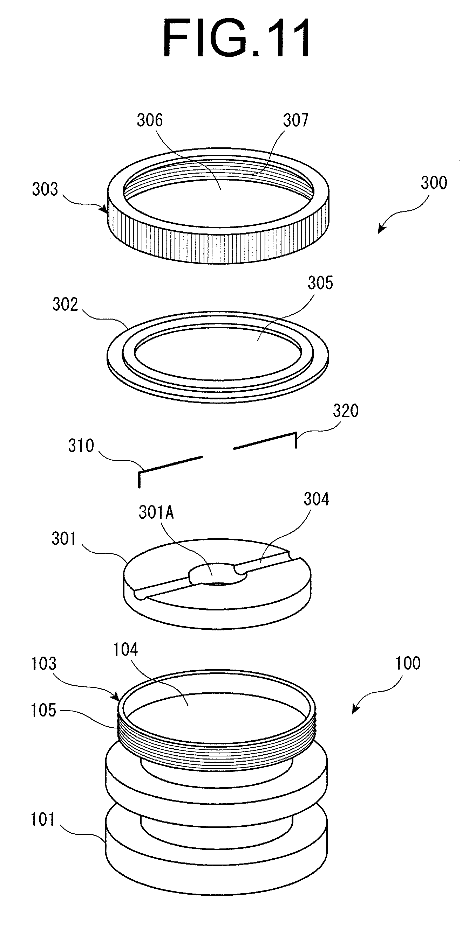

FIG. 10 is an entire schematic view of a surgical training device according to the third usage mode of the invention. FIG. 11 is an exploded perspective view showing outlines of a support mount and a fix hold portion in the surgical training device as shown in FIG. 10.

In addition, the surgical training device 30 of this usage mode has the same configuration as in the first usage mode except a fix hold portion (first holder and second holder). Therefore, the same portions as in the first usage mode shown in FIG. 1 are assigned the same reference numerals to omit specific descriptions thereof.

As shown in FIG. 10, the surgical training device 30 of this usage mode is comprised of the support mount 100, and a fix hold portion 300, provided on the upper face of the support mount 100, provided with a first hold shaft portion (fix holder; first holder) 310 that holds the first pseudo blood vessel (or artificial blood vessel) BV1 in a fixed manner, and a second hold shaft portion (fix holder; second holder) 320 that holds the second pseudo blood vessel (or artificial blood vessel) BV2 in a fixed manner. Herein, each of the first hold shaft portion 310 that holds the first pseudo blood vessel BV1 in a fixed manner and the second hold shaft portion 320 that holds the second pseudo blood vessel BV2 in a fixed manner maybeprovidedwithafixmaterial (dotted-lineportionsofreference numerals 310 and 320 in FIG. 10), for example, in the shape of a U or an L to be inserted into the inner surface of each pseudo blood vessel to hold so that the first pseudo blood vessel BV1 and the second blood vessel BV2 do not shift on the surface of the hold member 301 easily, respectively.

As shown in FIG. 11, the fix hold portion 300 is comprised of the hold member 301 that holds the first and second pseudo blood vessels BV1, BV2, the first hold shaft portion 310 that is inserted into the first pseudo blood vessel BV1, the second hold shaft portion 320 that is inserted into the second pseudo blood portion BV2, a fix portion 303 to fix the hold member 301 to the fix holder installation portion 103 of the support mount 100, and a pressing plate 302 that is positioned in between the hold member 301 and fix portion 303 to prevent the hold member 301 and the pseudo blood vessels BV1, BV2 from being misaligned, and the like.

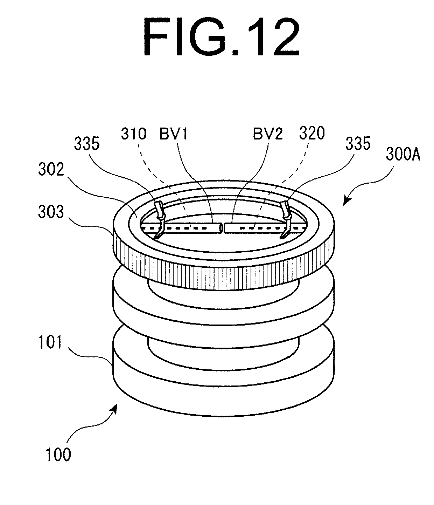

The hold member 301 is preferably comprised of a member having elasticity, and on its surface is formed a groove 304 to place the first and secondpseudo blood vessels BV1. BV2. In addition, to support pseudo blood vessels (or artificial blood vessels) of various sizes, as the groove provided in the hold member 301, a plurality of grooves with different groove widths and groove depths may be provided. Then, an opening 301A is provided substantially in the center portion of the hold member 301 not to interfere in anastomosis training. In addition, as shown in FIG. 12, clips 335 may be substituted for the hold member to hold the pseudo blood vessels BV1, BV2.

The first hold shaft portion (fix holder; first holder) 310 and second hold shaft portion (fix holder; second holder) 320 are formed of shaft members which are inserted in the holes of the pseudo blood vessels BV1, BV2 and thereby hold the pseudo blood vessels BV1, BV2, respectively. For the hold shaft portions 310, 320, it is suitable to select and use a shaft portion with the most suitable thickness for the thickness of a pseudo blood vessel to use as appropriate. As shown in FIG. 11, it is suitable that each of the hold shaft portions 310, 320 has substantially the shape of an L. By this means, the position is capable of being fixed easily by inserting each portion in between the hold member 301 and the inside of the fix holder installation portion 103. In addition, the hold shaft portions 310, 320 may be of configurations for enabling the portions to be fixed to the pressing plate 302 or the fix holder installation portion 103.

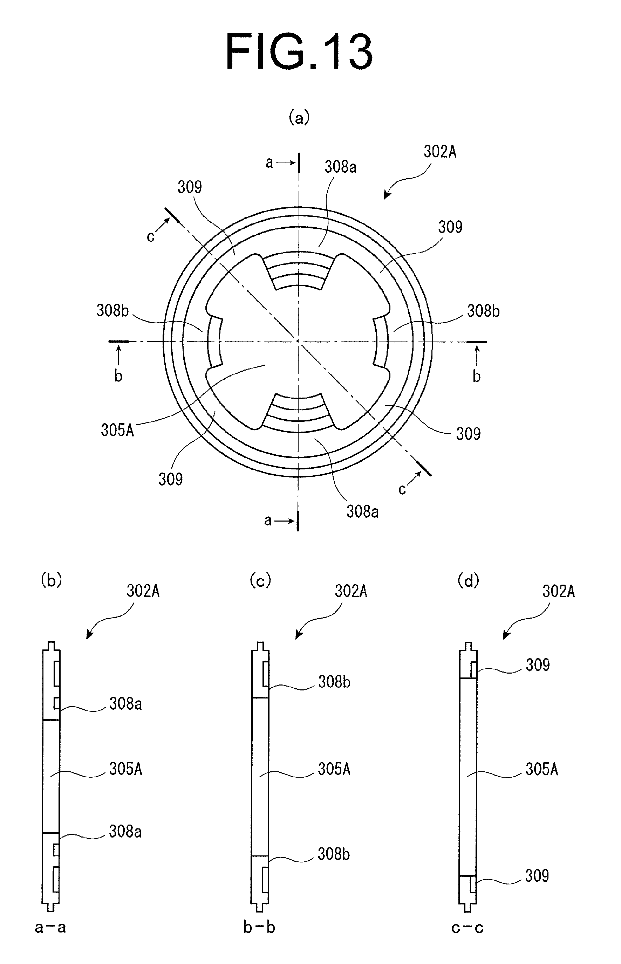

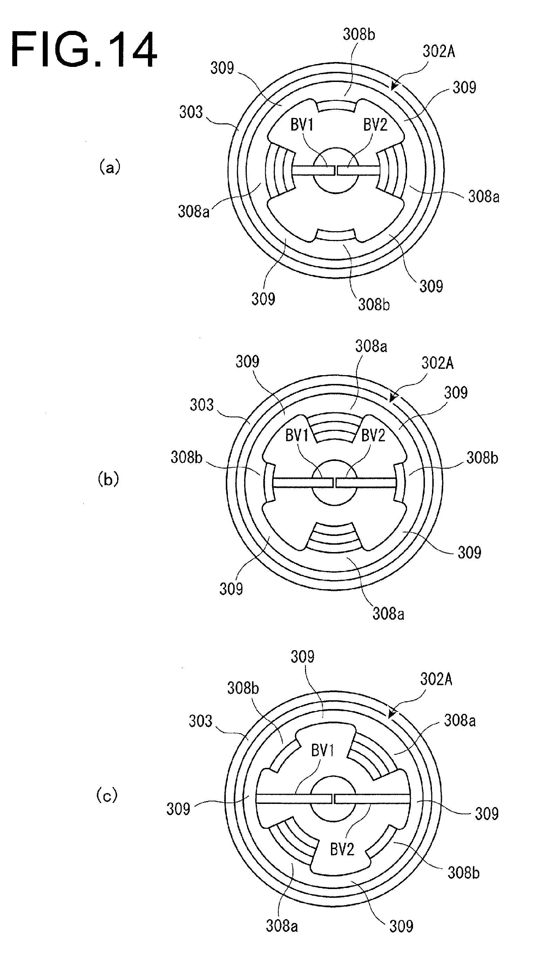

The pressing plate 302 is a plate-shaped member that presses the circumferential portion of the hold member 301, and the end portions of the pseudo blood vessels BV1, BV2, and has an opening 305. In addition, the pressing plate 302 may be the shape having protrusion portions 308 as shown in FIG. 13. Herein, FIG. 13(a) is a top view of the pressing plate 302A, FIG. 13(b) is a sectional view taken along the line a-a of FIG. 13(a), FIG. 13(c) is a sectional view taken along the line b-b of FIG. 13(a), and FIG. 13(d) is a sectional view taken along the line c-c of FIG. 13(a). Herein, an example of providing two groups (308a, 308b) of protrusion portions 308 with different lengths is shown in the figure, and further, a plurality of groups or only one group may be provided. The protrusion portions 308 are provided to be line symmetry with respect to the diameter. FIG. 14 contains views to explain usage examples of the pressing plate 302A. FIG. 14(a) shows a case of pressing the pseudo blood vessels BV1, BV2 with the large protrusion portions 308a, FIG. 14(b) shows a case of pressing the pseudo blood vessels BV1, BV2 with the small protrusion portions 308b, and FIG. 14(c) shows a case of pressing the pseudo blood vessels BV1, BV2 with inter-protrusion portions 309. Thus, in the case of providing two sets (308a, 308b) of protrusion portions 308 with different lengths, it is possible to press the pseudo blood vessels BV1, BV2 in three different positions. As shown in the figures, by forming the protrusion portions 308, even in the case where the lengths of the pseudo blood vessels BV1, BV2 are short, it is possible to reliably press the end portions.

The fix portion 303 has an opening 306 on the top, while in the inner circumference surface is formed a screw portion 307 to be screwed with the screw portion 105 of the support mount 100. Accordingly, by pressing the circumferential portion of the hold member 301 and the end portions of the pseudo blood vessels BV1, BV2 together with the pressing plate 302, while exposing the pseudo blood vessels BV1, BV2 from the opening 306, it is possible to fix to the support mount 100.

Thus, it is possible to configure the surgical training device 30 of this usage mode, only by adding the slight modification to the configuration of the fix holder 110 of the surgical training device 10 shown in the first usage mode.

Described below is the case of performing vascular anastomosis training using the surgical training device 30 with the above-mentioned configuration.

First, the first pseudo blood vessel BV1 and the second pseudo blood vessel BV2 are respectively inserted into the first hold shaft portion 310 and the second hold shaft portion 320, thereby set in the hold member 301, and after fitting the hold member 301 into the concave portion 104 of the support mount 100 together with the hold shaft portions 310, 320, are fixed with the pressing plate 302 and fix portion 303. At this point, the first pseudo blood vessel BV1 and the second pseudo blood vessel BV2 are fixed while protruding through the front ends of the hold shaft portions 310, 320 in predetermined lengths, respectively.

By this means, as shown in FIG. 9, the first pseudo blood vessel BV1 and the second pseudo blood vessel BV2 are anastomosed with the suture 51 and the surgical needle 52.

Thus, according to the aforementioned third usage mode, the support mount 100 and the fix holders 310, 320 are integrated, and it is thereby possible to configure the surgical training device that is compact and easy to use, and to perform training in end-end anastomosis with ease.

In the above-mentioned description, the usage modes of the invention are described, but the invention is not limited to the above-mentioned usage modes, and various modifications thereof can be made based on the subject matter of the invention, and the modifications are not excluded from the scope of the invention.

### Industrial Applicability

The present invention relates to the surgical training device used in training in surgeries, particularly, vascular anastomosis, and has industrial applicability.

### Description of Symbols

- 10, 20, 30: Surgical training device
- 100: Support mount
- 101: Support portion
- 102: Coupling portion
- 103: Fix holder installation portion
- 104: Concave portion
- 105: Screw portion
- 110: First holder (fix holder)
- 111,: 111A Hold member
- 112: Pressing plate
- 113: Fix portion
- 114, 114a, 114b: Groove
- 115, 116: Opening
- 117: Screw portion
- 120: Second holder (movable holder)
- 121: Fix portion
- 122: Flexible arm portion
- 123: Hold shaft portion
- 124: Connection portion
- 125: Clip
- 126: Connector member
- 126A: Ball
- 126B: Shaft
- 127: Cylindrical member
- 210: First holder (first movable holder)
- 211: Fix portion
- 212: Flexible arm portion
- 213: Hold shaft portion
- 214: Connection portion
- 215: Clip
- BV1: First pseudo blood vessel (or artificial blood vessel)
- BV2: Second pseudo blood vessel (or artificial blood vessel)
- 300: Fix hold portion
- 301: Hold member
- 301A: Opening
- 302: Pressing plate
- 303: Fix portion
- 304: Groove
- 305, 306: Opening
- 307: Screw portion
- 308, 308a, 308b: Protrusion portion
- 309: Inter-protrusion portion
- 310: First hold shaft portion (fix holder; first holder)
- 320: Second hold shaft portion (fix holder; second holder)
- 335: Clip

## Claims

1. A surgical training device to perform simulations and practices of vascular anastomosis, comprising:
a support mount of the surgical training device;
a first holder disposed on the support mount to hold a first pseudo blood vessel; and
a second holder disposed on the support mount to hold a second pseudo blood vessel to be anastomosed to the first pseudo blood vessel,
wherein the first holder holds the first pseudo blood vessel to enable a front end portion of the first pseudo blood vessel to be placed in an arbitrary position in the second pseudo blood vessel held by the second holder and in an arbitrary direction with respect to the axis direction of the second pseudo blood vessel.

2. The surgical training device according to claim 1, wherein the first holder has a hold member, formed on the top surface of the support mount, having a single or a plurality of grooves with different diameters to fix and hold the first pseudo blood vessel.

3. The surgical training device according to claim 1, wherein the first holder is a multijoint movable holder that is coupled to the support mount and that enables the first pseudo blood vessel to be shifted to an arbitrary position while holding the first pseudo blood vessel in the axis direction of the first pseudo blood vessel.

4. The surgical training device according to claim 2 or 3, wherein the second holder is a multijoint movable holder that is coupled to the support mount and that enables the second pseudo blood vessel to be shifted arbitrarily while holding the second pseudo blood vessel in the blood vessel axis direction of the second pseudo blood vessel.

5. The surgical training device according to claim 1, wherein the first holder and the second holder are fix holders formed in an upper portion of the support mount to fix and hold the first pseudo blood vessel and the second pseudo blood vessel in the axis direction of each blood vessel, respectively.

6. The surgical training device according to claim 3 or 4, wherein the movable holder has a flexible armportion comprised of a structure for alternately coupling a plurality of connector members each in the shape of a dumbbell having balls at opposite ends of a cylinder, and a plurality of cylindrical bodies into which the balls of the connector members are fitted to hold the balls rotatably.
